# EUROPEAN PATENT SPECIFICATION

(11) **EP 2 785 237 B1**
(45) Date of publication and mention of the grant of the patent: **30.09.2020**
(21) Application number: 12809851.4
(22) Date of filing: 12.11.2012
(51) Int. Cl.: A61B 34/10, A61B 1/313

(54) **SURGICAL PORT LOCALIZATION**
LOKALISIERUNG CHIRURGISCHER PORTS
LOCALISATION D'UN ORIFICE CHIRURGICAL

(30) Priority: 03.12.2011 US 201161566614 P
(43) Date of publication of application: 08.10.2014
(73) Proprietor: Koninklijke Philips N.V., 5656 AG Eindhoven (NL)
(72) Inventor: POPOVIC, Aleksandra, 5656AE Eindhoven (NL); ELHAWARY, Haytham, 5656AE Eindhoven (NL); CHAN, Raymond, 5656AE Eindhoven (NL); MANZKE, Robert, 5656AE Eindhoven (NL)
(74) Representative: Philips Intellectual Property & Standards
(86) International application number: PCT/IB2012/056350
(87) International publication number: WO 2013/080070

(56) References cited:
- US-A1- 2006 173 438
- US-A1- 2011 202 069
- US-B2- 7 930 065

## Description

### FIELD OF THE INVENTION

The invention relates to the field of medical imaging and more particularly to an apparatus for locating a surgical port with respect to an endoscopic device.

### BACKGROUND

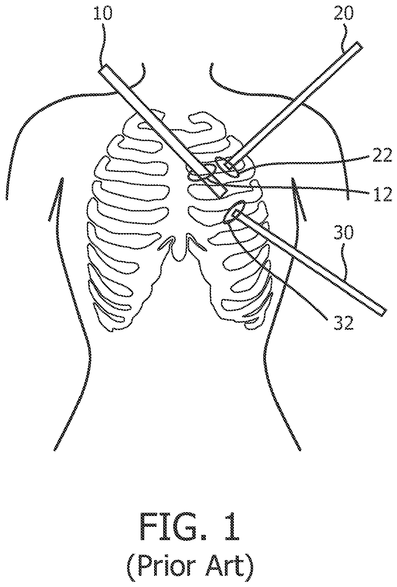

Minimally invasive surgery is performed using elongated instruments 10, 20, 30 inserted into a patient's body through small ports 12, 22, 32, as shown in Fig. 1. The number of ports may vary from one port to three or more ports. In many procedures one of the instruments inserted into a patient's body is an endoscope, providing real-time visualization of a body cavity where the procedure is performed. Placement of the ports plays an important role in the outcome of the surgery. Instruments positioned in a port at a suboptimal location port may not be able to reach all areas of interest.

In a standard clinical practice, the ports are located using guidelines published by professional associations, hospitals, or manufacturers of surgical equipment. The guidelines define entry points with respect to some well-known anatomical landmarks. For example, in cardiac surgery ports are located by defining anatomical landmarks, such as: ribs, sternum, and nipple, together with defining distances from these landmarks. This kind of guidance does not take into account variability in anatomy (both between patients and between pre-operative versus intra-operative anatomy), patient size, specificity of patient's disease or condition, and the like. A more patient-specific port placement can be beneficial, minimizing access-related tissue trauma and procedure times while improving outcomes.

After the instruments are inserted into the body cavity through the ports, they can be moved in four degrees of freedom (DOF): two angles of rotation pivoting around the port (fulcrum point), insertion, and rotation about the instruments longitudinal axis. The angles of rotation are rotation about imaginary axes that are perpendicular to each other and to the longitudinal axis of the instrument. Whereas rotation and insertion are intuitive and easy to use, mapping between the angles of rotation and an endoscope view of the body cavity is less intuitive, takes a long time to learn, and, as studies show, is the biggest hurdle to hand-eye coordination in minimally invasive surgery.

Much of the research on improving port placement is focused on developing computer algorithms to compute optimal ports for instruments based on preoperative 3D medical images. The subsequent translation of these optimized plans into an operating room is focused on using standard tool tracking technologies known from surgical navigation. However, tool tracking on preoperative 3D images does not take into account that spatial relationships between anatomical landmarks may significantly change from preoperative images to intraoperative situation. For example, in minimally invasive surgery CO2 is introduced into the natural cavity (such as chest or abdomen) to expand the patient and provide a larger space for instruments. After introduction of the CO2, the spatial arrangement between outer surface of the patient (e.g. chest) and organs (such as heart) would change. Thus, the preoperative planning of port placement would not be useful in the new arrangement.

Publ. Pat. Appl. No. US 2011/202069 A1 by Giuseppe M. Prisco et al. is directed to an apparatus for three-dimensional measurements in a fixed world reference frame. A reference fixture includes a joint and a joint tracker to track motion of the joint. A surgical instrument is connected to the reference fixture by a tether. A shape sensor extends from the reference fixture, through the tether, and into the surgical instrument. The shape sensor is substantially free of twist. Information from the joint tracker and the shape sensor are used to register three-dimensional information to a fixed world reference frame.

Pat. No. US7,930,065 to David Q. Larkin et al. is directed to a robotic surgery system with position sensors using fiber Bragg gratings to determine the position of a joint of an articulatable arm.

### SUMMARY

A device and system are provided for placing a port for a surgical tool relative to real-time anatomical data. The invention is defined by the appended claims.

According to one aspect of the present invention, a device is provided for locating a port for a surgical tool relative to real-time anatomical data from an endoscope. The device comprises: a port localization apparatus affixed to the endoscope at a predetermined point and locating a port at a known location relative to the endoscope.

The device further comprises a processor. The processor is configured to determine the port location in an image space of the endoscope, to determine a projection of an instrument through the port location onto an image from the endoscope, and to overlay a virtual representation of the instrument onto the endoscope image corresponding to the potential port location.

According to one embodiment the device further comprises a positioning and orientation apparatus operably connected to the port localization apparatus. The positioning and orientation apparatus is adapted to be manipulated to providing angles of projection at the port location for a projection of an instrument onto the endoscope image. The positioning and orientation apparatus captures the angles of projection and determining the location of the projection on the endoscope image.

According to one embodiment, the port localization apparatus is a shape sensing tether and the positioning and orientation apparatus is a stylus.

According to one embodiment, the port localization apparatus is at least one rigid member and the positioning and orientation apparatus is at least one joint.

According to another aspect of the present invention, a system is provided for locating a port for a surgical tool relative to an endoscope. The system comprises: a processor, a memory operably connected with the processor, an endoscope providing an endoscope image, a port localization apparatus affixed to the endoscope at a predetermined point and locating a port at a known location relative to the endoscope, and a program of instruction encoded on the memory and executed by the processor to determine the location of the port.

According to one embodiment, the program of instruction executed by the processor: determines the port location in an image space of the endoscope, determines a projection of an instrument through the port location onto an image from the endoscope, and overlays a representation of the instrument onto the endoscope image corresponding to the potential port location.

According to one embodiment, the system further comprises a positioning and orientation apparatus operably connected to the port localization apparatus. The positioning and orientation apparatus is adapted to be manipulated to providing angles of projection at the port location for a projection of an instrument onto the endoscope image. The positioning and orientation apparatus captures the angles of projection and the port localization program of instruction determines the location of the projection on the endoscope image.

According to one embodiment, the port localization apparatus is a shape sensing tether and the positioning and orientation apparatus is a stylus.

According to one embodiment, the port localization apparatus is at least one rigid member and the positioning and orientation apparatus is at least one joint connected to the at least one rigid member and having an encoder measuring an angle of the joint.

### BRIEF DESCRIPTION OF THE DRAWINGS

The features and advantages of the invention will be more clearly understood from the following detailed description of the preferred embodiments when read in connection with the accompanying drawing. Included in the drawing are the following figures:
Fig. 1 is an isometric view of a minimally invasive procedure according to the prior art;
Fig. 2 is a sectional view of a minimally invasive procedure using a device for locating a port for a surgical tool relative to real-time anatomical data from an endoscope according to an embodiment of the present invention;
Fig. 3 is an endoscope image from the procedure of Fig. 2;
Fig. 4 is a block diagram of a system for locating a port for a surgical tool relative to real-time anatomical data from an endoscope according to an embodiment of the present invention;
Fig. 5 is a flow diagram of a method for locating a port for a surgical tool relative to real-time anatomical data from an endoscope according to an embodiment of the present invention;
Fig. 6 is a flow diagram of a method for using a port localization apparatus to identify an optimal location for a port for a surgical instrument relative to real-time anatomical data according to an embodiment of the present invention;
Fig. 7 is a representation of a device for locating a port for a surgical tool relative to real-time anatomical data from an endoscope according to an embodiment of the present invention;
Fig. 8 is side view of a device for locating a port for a surgical tool relative to real-time anatomical data from an endoscope according to another embodiment of the present invention;
Fig. 9 is a front view of the device of Fig. 8; and
Fig. 10 is a representation of the device of Figs. 8 and 9 showing an alternative attachment to the endoscope according to an embodiment of the present invention.

### DETAILED DESCRIPTION

The present invention provides a device and system for locating a port for a surgical tool relative to real-time anatomical data from an endoscope.

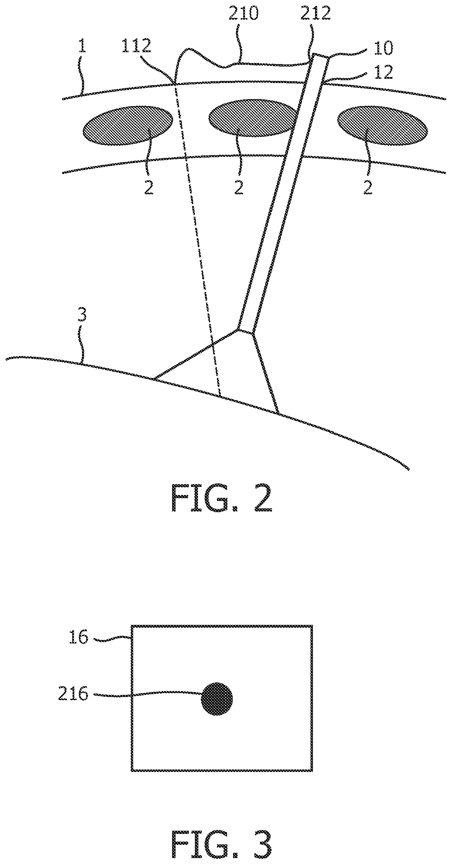

Fig. 2 shows a device for locating a port for a surgical tool relative to real-time anatomical data from an endoscope. According to one embodiment, an endoscope 10 is inserted through a standard port 12. The standard port is located using existing techniques. The endoscope provides an image 16 of anatomical features. In the illustrated embodiment, the endoscope 10 is inserted through the port 212 in the skin 1, between the ribs 2, and into the thoracic cavity to provide an image 16 of the heart 3, as shown in Fig. 3.

Returning to Fig. 2, an optical shape sensing fiber 210 (OSS) is connected to the endoscope 10 at a known anchor point 212. In shape sensing, properties of a fiber optic cable are used to determine the 3-dimensional shape of the fiber optic cable. Shape sensing based on fiber optics is known in the art. One approach is based on fiber optic Bragg grating sensors. A fiber optic Bragg grating (FBG) is a short segment of optical fiber that reflects particular wavelengths of light and transmits other wavelengths of light. This is achieved by adding a periodic variation of the refractive index in the fiber core, which generates a wavelength-specific dielectric mirror. An FBG can therefore be used as an inline optical filter to block transmission of certain wavelengths or as a selective specific wavelength reflector - the specific wavelength being the Bragg wavelength.

The Bragg wavelength is sensitive to strain as well as temperature. This means that Bragg gratings can be used as sensing elements in fiber optic sensors. In an FBG sensor, the measurand (e.g., strain) causes a shift in the Bragg wavelength.

One of the main advantages of FBG's is that various sensor elements can be distributed over the length of a fiber. Incorporating 3 or more cores with various sensors (gauges) along the length of a fiber that is embedded in a structure allows for the 3-dimensional form of the structure to be precisely determined. A plurality of FBG sensors are positioned along the length of the fiber (e.g., in 3 or more sensing cores). From the Bragg wavelength shift, the strain can be measured at each FBG. From the strain measurements, at various positions, the curvature at these positions can be inferred. From the plurality of measured positions, the total 3-dimensional form is determined.

As an alternative to FBG, the inherent backscatter in conventional optical fiber can be exploited for shape sensing. One such approach is to use Raleigh scatter in standard, single-mode communications fiber. Raleigh scatter occurs as a result of random fluctuations of the index of refraction in the fiber core. These random fluctuations can be modeled as a Bragg grating with random variation of amplitude and phase along the grating length. By using this effect in 3 or more cores running within a single length of multicore fiber, the 3D shape and dynamics of the surface of interest is trackable.

With the optical fiber connected to the endoscope at a known anchor point, the spatial relationship to the endoscope image 16 is established using two mechanical properties of the mounting: (1) the distance from the center axis of the endoscope to the anchor point (d); and (2) the distance from the anchor point to the endoscope image plane (1) (i.e., the focal length of the endoscope + the distance from the lens of the endoscope to the anchor point along the axis of the endoscope).

These two values: (d) and (1) are used to translate the coordinate system of the fiber to the coordinate system of the endoscopic image so that the measured shape of the optic fiber beyond the anchor point 212 can be calculated in the endoscope coordinate system. Thus, a potential port location 112 can be contacted with the free end of the optic fiber 210 to locate it relative to the endoscopic image 16. Moreover, the shape of the optic fiber can be extended using a 3D modeling application to project a virtual surgical tool onto the plane of the endoscopic image 16. A representation of the surgical tool 216, such as a virtual dot, is then overlaid onto the endoscopic image 16, and used to determine whether or not the proposed port location 112 is optimal.

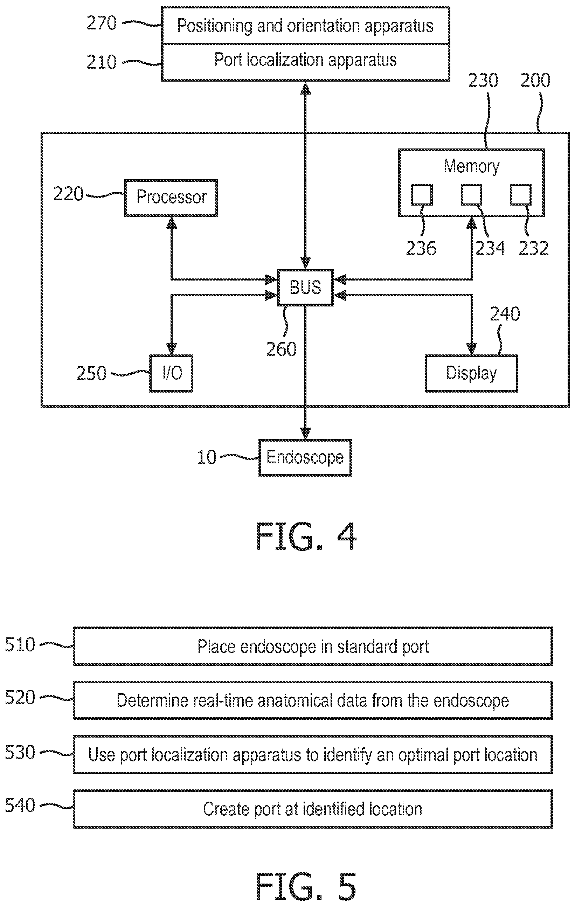

Fig. 4 is a block diagram of a system for placing a port for a surgical tool relative to real-time anatomical data according to an embodiment of the present invention. The system comprises: a port localization apparatus 210, a processing system 200, and an endoscope 10. The processing system 200 comprises one or more processors 220 operably connected with one or more memories 230. They may be connected, for example, through a system bus 260. It should be understood that other suitable architectures are also possible within the scope of the present invention. The processor 220 may be any suitable processor, such as one or more microprocessors. The memory 230 may be any suitable memory, including but not limited to: RAM, ROM, an internal hard drive, a disk drive, a USB flash drive, or any other memory device suitable for storing program code.

The memory 230 has encoded on it a localization program of instruction 232 executed by the processor 220 to locate a port for a surgical tool relative to real-time anatomical data from the endoscope 10. The system also comprises one or more displays 240 for presenting a user interface with endoscope data and one or more input and/or output devices 250 for entering user inputs, for example.

The memory 230 may also have endoscope program instructions 234 for presenting an endoscope image 16 on the display 240, and modeling program instructions 236 for modeling and projecting a virtual representation 216 of a surgical tool through a potential port location onto the endoscope image 16.

Fig. 5 is a flow diagram of a method for locating a port for a surgical tool relative to real-time anatomical data from an endoscope according to an embodiment of the present invention. A surgeon places an endoscope 10 in a standard port 12 (Step 510). That is, the surgeon locates the port using a standard port locating procedure.

The processor 220 executes the endoscope program instructions 232 to determine real-time anatomical data from the endoscope 10 (Step 520). The real-time anatomical data may be the digitized endoscopic image 16, such as the coordinates and intensity values for each pixel of the image.

The port localization program instructions 232 executed by the processor 220 uses the port localization apparatus 210 to identify an optimal port location (Step 530). Then, the surgeon creates a port at the determined location (Step 540).

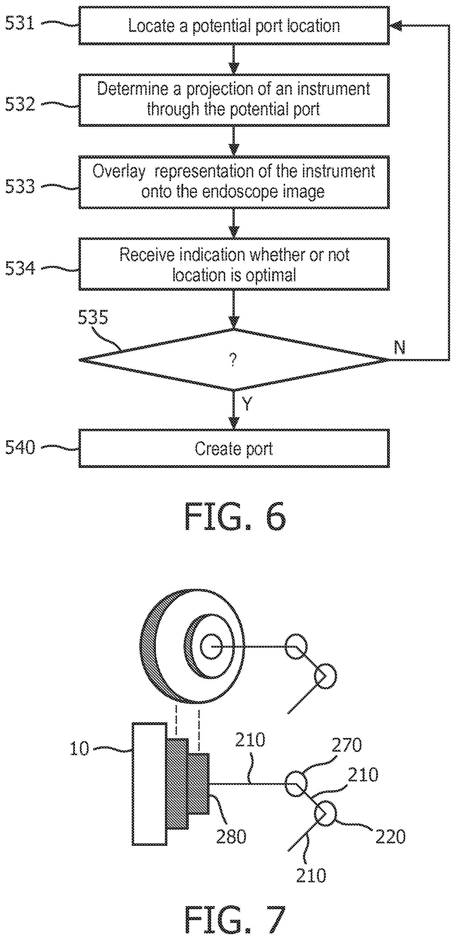

According to one embodiment, the port localization apparatus 210 comprises a tether with an optical shape-sensing fiber which is fixed to the endoscope at a known anchor point 212. As shown in Fig. 6, the free end of the optical shape-sensing fiber is positioned at a potential port location (Step 531). Then, the port localization program instructions 232 executed by the processor 220 projects a virtual tool through the potential port location onto the endoscopic image 16 (Step 532). The virtual tool is projected by translating the location of the potential port to endoscopic image coordinates, then extending a line from the potential port location onto the endoscopic image. The line is extended at predetermined tool angles pivoted about the potential port location, such as parallel to the endoscope, perpendicular to the surface of the patient's skin, or at any other predetermined angle. The projection is used to determine whether or not the potential port location is optimal. This determination may be made by the surgeon or by the port localization program instructions 232 executed by the processor 220.

If the potential port location is optimal, then the surgeon creates a port at the determined location (Step 540).

According to the invention, a representation of the projection of the surgical tool is overlaid on the endoscopic image 16 (Step 533). Based on the position of the representation, a determination is made of whether or not the potential port location is optimal. This determination can be made by the surgeon after looking at the position of the representation of the tool, or by the port localization program instructions 232 executed by the processor 220 using a modeling application. The port localization program instructions 232 executed by the processor 220 then receives the indication of whether or not the location is optimal (Step 534). The port localization program instructions 232 executed by the processor 220 may iteratively test potential port locations until an optimal location is located (Step 535).

According to another embodiment, a virtual line is determined directly from the free end of the optic fiber 210 to the center of the endoscope image 16 and the resulting tool angles at the potential port location are determined using a modeling application. Then a determination of whether or not the potential port location is optimal is made based on the determined tool angles.

According to one embodiment, a positioning and orientation apparatus 270 is connected to the localization apparatus 210 to project the projection of the instrument onto the endoscope image 16. The positioning and orientation apparatus 270 captures the angles of projection and determines the location of the projection on the endoscope image corresponding to the captured angles. The positioning and orientation apparatus 270 may be, for example, a stylus simulating the handle of a surgical tool. The optic fiber 210 may be fixed in the stylus, such as through a longitudinal opening, such that the end of the optic fiber (captured in the stylus) is held at the tool angles relative to the potential port location. In this embodiment, the angles are captured by shape sensing of the optic fiber.

The surgeon holds the positioning and orientation apparatus 270 at a potential port location and manipulates it to simulate the intended procedure. As described in the previous embodiment, the port locating program of instruction 232 translates the potential port location to endoscope coordinates using the known anchor point and the determined shape of the optic fiber. The modeling program instructions 236 extend a virtual tool from the potential port location along the angles defined by the end of the optic fiber held in the stylus. The projection of the tool is then overlaid onto the endoscope image 16 so that the surgeon can see the range of motion corresponding to the potential port location and determine whether or not the potential port location is optimal.

Fig. 7 shows a representation of a device for locating a port for a surgical tool relative to real-time anatomical data from an endoscope according to another embodiment of the present invention. In this embodiment, the port localization apparatus 210 is at least one rigid member such as an arm, a beam, or the like. The positioning and orientation apparatus 270 is at least one hinged joint with an encoder 280 that provides the angle of the joint. The angle can be provided as an electronic signal or it can be read off of the encoder 280 and entered manually to the port location program instructions 232. As with the optic fiber, the rigid port localization apparatus 210 has a known anchor point on the endoscope. Also, the length of the port localization apparatus 210 is known. The modeling program instructions can determine the location of the end of the port localization apparatus, and the potential port location, from the known anchor point, the arm length, and the joint angles.

According to one embodiment, the joints 270 are motorized to manipulate a port localization apparatus, or even a surgical tool, during a procedure by driving the joints to angles corresponding to a desired location for automatic positioning. In this embodiment, the surgeon may input a location on the endoscope image to automatically position a tool.

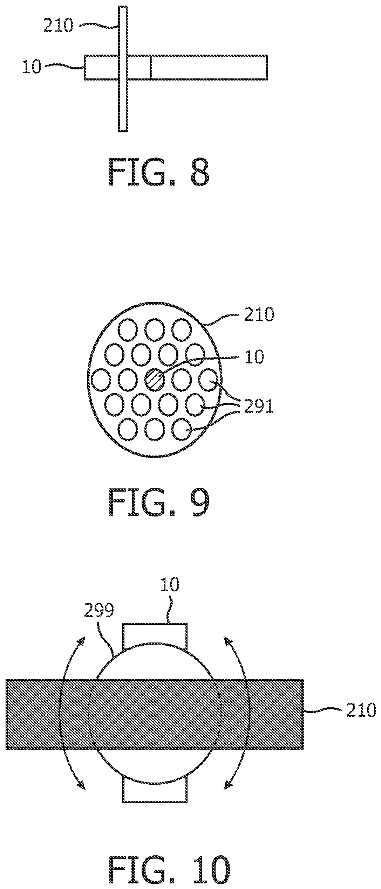

Figs. 8 and 9 show a port localization apparatus 210 according to another embodiment of the present invention. In this embodiment, the port localization apparatus 210 is a rigid disk mounted on the endoscope 10 at a center hole. The disk is provided with a plurality of holes 291 which are numbered. Each hole locates a potential port location. Because the disk is rigid, and the holes are a known distance from the center of the endoscope at a known orientation, the holes 291 can be translated into endoscope coordinates, and virtual tools can be projected from the corresponding potential port locations onto the endoscopic image 16. Representations of these virtual tools, such as numbers corresponding to the numbered holes are then overlaid onto the endoscope image 16. An optimal port location may then be selected based on the location of the corresponding virtual tool projection.

As shown in Fig. 10, the disk port localization apparatus 210 may be attached to the endoscope with a spherical joint 299 to allow the disk to rotate relative to the endoscope 10 so that the virtual tools may be projected at an angle to the potential port location. The spherical joint may be equipped with an encoder to provide the angle of rotation of the disk.

The invention can take the form of an embodiment containing both hardware and software elements. In an exemplary embodiment, the invention is implemented in software, which includes but is not limited to firmware, resident software, microcode, etc.

Furthermore, the invention may take the form of a computer program product accessible from a computer-usable or computer-readable medium providing program code for use by or in connection with a computer or any instruction execution system or device. For the purposes of this description, a computer-usable or computer readable medium may be any apparatus that can contain or store the program for use by or in connection with the instruction execution system, apparatus, or device.

The foregoing method may be realized by a program product comprising a machine -readable medium having a machine-executable program of instructions, which when executed by a machine, such as a computer, performs the steps of the method. This program product may be stored on any of a variety of known machine-readable medium, including but not limited to compact discs, floppy discs, USB memory devices, and the like.

The medium can be an electronic, magnetic, optical, electromagnetic, infrared, or semiconductor system (or apparatus or device). Examples of a computer-readable medium include a semiconductor or solid state memory, magnetic tape, a removable computer diskette, a random access memory (RAM), a read-only memory (ROM), a rigid magnetic disk an optical disk. Current examples of optical disks include compact disk-read only memory (CD-ROM), compact disk-read/write (CD-R/W) and DVD.

The preceding description and accompanying drawing are intended to be illustrative and not limiting of the invention.

## Claims

1. A device for locating a port for a surgical tool relative to real-time anatomical data from an endoscope, comprising:
a port localization apparatus (210) adapted to be affixed to the endoscope (10) at a predetermined anchor point, the port localization apparatus being adapted to define a potential location for the port at a known spatial relationship to the endoscope; and
a processor (220),
the device being **characterised in that** the processor is adapted:
to determine the potential port location in an image space of the endoscope;
to determine a projection (216) of an instrument through the potential port location onto an image from the endoscope; and
to overlay a representation of the instrument onto the endoscope image corresponding to the potential port location.

2. The device of claim 1, further comprising a positioning and orientation apparatus (270) operably connected to the port localization apparatus (210), the positioning and orientation apparatus being adapted to be manipulated to providing angles of projection at the port location for a projection of an instrument onto the endoscope image, the positioning and orientation apparatus being adapted to capture the angles of projection and being adapted to determine the location of the projection on the endoscope image.

3. The device of claim 2, wherein the port localization apparatus (210) is a shape sensing tether and the positioning and orientation apparatus is a stylus.

4. The device of claim 1, wherein the port localization apparatus is at least one rigid member and the positioning and orientation apparatus is at least one joint connected to the at least one rigid member and having an encoder (280) adapted to measure an angle of the joint.

5. A system for locating a port for a surgical tool relative to an endoscope, comprising:
the device of claim 1;
a memory (230) operably connected with the processor;
an endoscope (10) providing an endoscope image (16); and
a program of instruction encoded on the memory and when executed by the processor, the program of instruction is adapted to determine the location of the port.

6. The system of claim 5, wherein the program of instruction (232) executed by the processor (220):
determines the port location in an image space of the endoscope;
determines a projection of an instrument through the port location onto an image from the endoscope; and
overlays a representation of the instrument onto the endoscope image corresponding to the potential port location.

7. The system of claim 6, further comprising a positioning and orientation apparatus (270) operably connected to the port localization apparatus, the positioning and orientation apparatus being adapted to be manipulated to providing angles of projection at the port location for a projection (216) of an instrument onto the endoscope image (16), the positioning and orientation apparatus being adapted to capture the angles of projection and being adapted to determine the location of the projection on the endoscope image.

8. The system of claim 7, wherein the port localization apparatus (210) is a shape sensing tether and the positioning and orientation apparatus is a stylus.

9. The system of claim 7, wherein the port localization apparatus (210) is at least one rigid member and the positioning and orientation apparatus is at least one joint connected to the at least one rigid member and having an encoder (280) adapted to measure an angle of the joint.

10. A computer program product comprising a computer-readable storage device (230) having encoded thereon a program of instruction (232) executable by a computer processor (220) to place a port for a surgical tool relative to real-time anatomical data, the program of instruction comprising:
program instructions for determining real-time anatomical data from an image from an endoscope; **characterized in that** the
program instructions are adapted for using the port localization apparatus of the device of claim 1 to identify an optimal location for an instrument port relative to the image from the endoscope,
the computer program product further comprising:
program instructions for locating a potential port location;
program instructions for determining a projection of an instrument through the potential port location onto the plane of the endoscope image;
program instructions for overlaying a representation of the instrument onto the endoscope image corresponding to the potential port location; and
program instructions for receiving an indication of whether or not the potential port location is an optimal port location;
wherein the program instructions are encoded on the computer-readable storage device.

## Patentansprüche

1. Vorrichtung zum Lokalisieren eines Ports für ein chirurgisches Werkzeug relativ zu anatomischen Echtzeitdaten von einem Endoskop, umfassend:
eine Portlokalisierungsvorrichtung (210), die angepasst ist, um an einem vorbestimmten Ankerpunkt am Endoskop (10) angebracht zu werden, wobei die Portlokalisierungsvorrichtung angepasst ist, um eine potentielle Position für den Port in einer bekannten räumlichen Beziehung zum Endoskop zu definieren; und
einen Prozessor (220),
wobei die Vorrichtung **dadurch gekennzeichnet ist, dass** der Prozessor angepasst ist zum:
Bestimmen der potentiellen Portposition in einem Bildraum des Endoskops;
Bestimmen einer Projektion (216) eines Instruments durch die potentielle Portposition auf ein Bild vom Endoskop; und
Überlagern einer Darstellung des Instruments auf das Endoskopbild, das der potentiellen Portposition entspricht.

2. Vorrichtung nach Anspruch 1, ferner umfassend eine Positionierungs- und Orientierungsvorrichtung (270), die funktionsfähig mit der Portlokalisierungsvorrichtung (210) verbunden ist, wobei die Positionierungs- und Orientierungsvorrichtung so angepasst ist, dass sie manipuliert werden kann, um Projektionswinkel an der Portposition für eine Projektion eines Instruments auf das Endoskopbild bereitzustellen, wobei die Positionierungs- und Orientierungsvorrichtung angepasst ist, um die Projektionswinkel zu erfassen, und angepasst ist, um die Position der Projektion auf dem Endoskopbild zu bestimmen.

3. Vorrichtung nach Anspruch 2, wobei die Portlokalisierungsvorrichtung (210) ein Formerfassungsband ist und die Positionierungs- und Orientierungsvorrichtung ein Stift ist.

4. Vorrichtung nach Anspruch 1, wobei die Portlokalisierungsvorrichtung mindestens ein starres Element ist, und die Positionierungs- und Orientierungsvorrichtung mindestens ein Gelenk ist, die mit dem mindestens einen starren Element verbunden ist, und einen Codierer (280) aufweist, der zum Messen eines Winkels des Gelenks angepasst ist.

5. System zum Lokalisieren eines Ports für ein chirurgisches Werkzeug relativ zu einem Endoskop, umfassend:
die Vorrichtung nach Anspruch 1;
einen Speicher (230), der funktionsfähig mit dem Prozessor verbunden ist;
ein Endoskop (10), das ein Endoskopbild (16) bereitstellt; und
ein Befehlsprogramm, das in dem Speicher codiert ist, und wenn es vom Prozessor ausgeführt wird, das Befehlsprogramm angepasst wird, um die Portposition zu bestimmen.

6. System nach Anspruch 5, wobei das von dem Prozessor (220) ausgeführte Befehlsprogramm (232):
bestimmt die Portposition in einem Bildraum des Endoskops;
bestimmt eine Projektion eines Instruments durch die Portposition auf ein Bild vom Endoskop; und
überlagert eine Darstellung des Instruments mit dem Endoskopbild, das der potentiellen Portposition entspricht.

7. System nach Anspruch 6, ferner umfassend eine Positionierungs- und Orientierungsvorrichtung (270), die funktionsfähig mit der Portlokalisierungsvorrichtung verbunden ist, wobei die Positionierungs- und Orientierungsvorrichtung so angepasst ist, dass sie manipuliert werden kann, um Projektionswinkel an der Portposition für eine Projektion (216) eines Instruments auf das Endoskopbild (16) bereitzustellen, wobei die Positionierungs- und Orientierungsvorrichtung angepasst ist, um die Projektionswinkel zu erfassen, und angepasst ist, um die Position der Projektion auf dem Endoskopbild zu bestimmen.

8. System nach Anspruch 7, wobei die Portlokalisierungsvorrichtung (210) ein Formerfassungsband ist und die Positionierungs- und Orientierungsvorrichtung ein Stift ist.

9. System nach Anspruch 7, wobei die Portlokalisierungsvorrichtung (210) mindestens ein starres Element ist und die Positionierungs- und Orientierungsvorrichtung mindestens ein Gelenk ist, die mit dem mindestens einen starren Element verbunden ist, und einen Codierer (280) aufweist, der zum Messen eines Winkels des Gelenks angepasst ist.

10. Computerprogrammprodukt, umfassend eine computerlesbare Speichervorrichtung (230), auf der ein von einem Computerprozessor (220) ausführbares Befehlsprogramm (232) codiert ist, um einen Port für ein chirurgisches Werkzeug in Bezug auf anatomische Echtzeitdaten zu platzieren, wobei das Befehlsprogramm umfasst:
Programmbefehle zum Bestimmen anatomischer Echtzeitdaten aus einem Bild von einem Endoskop; **dadurch gekennzeichnet,**
**dass** die Programmbefehle zur Verwendung der Portlokalisierungsvorrichtung der Vorrichtung nach Anspruch 1 angepasst sind, um eine optimale Position für einen Instrumentenport relativ zu dem Bild vom Endoskop zu identifizieren,
das Computerprogrammprodukt, ferner umfassend:
Programmbefehle zum Lokalisieren einer potentiellen Portposition;
Programmbefehle zum Bestimmen einer Projektion eines Instruments durch die potentielle Portposition auf die Ebene des Endoskopbildes;
Programmbefehle zum Überlagern einer Darstellung des Instruments auf das Endoskopbild, das der potentiellen Portposition entspricht; und
Programmbefehle zum Empfangen einer Anzeige, ob die potentielle Portposition eine optimale Portposition ist oder nicht;
wobei die Programmbefehle auf der computerlesbaren Speichervorrichtung codiert sind.

## Revendications

1. Dispositif pour localiser un orifice pour un outil chirurgical en fonction de données anatomiques en temps réel provenant d'un endoscope, comprenant:
un appareil de localisation d'orifice (210) adapté pour être fixé à l'endoscope (10) à un point d'ancrage prédéterminé, l'appareil de localisation de l'orifice étant adapté pour définir un emplacement potentiel pour l'orifice à une relation spatiale connue à l'endoscope; et
un processeur (220),
le dispositif étant **caractérisé en ce que** le processeur est adapté:
pour déterminer l'emplacement potentiel de l'orifice dans un espace d'image de l'endoscope;
pour déterminer une projection (216) d'un instrument à travers l'emplacement potentiel de l'orifice sur une image provenant de l'endoscope; et
pour superposer une représentation de l'instrument sur l'image de l'endoscope correspondant à l'emplacement potentiel de l'orifice.

2. Dispositif selon la revendication 1, comprenant en outre un appareil de positionnement et orientation (270) connecté de manière opérationnelle à l'appareil de localisation de l'orifice (210), l'appareil de positionnement et orientation étant adapté pour être manipulé pour fournir des angles de projection à l'emplacement de l'orifice pour une projection d'un instrument sur l'image de l'endoscope, l'appareil de positionnement et orientation étant adapté pour capturer les angles de projection et étant adapté pour déterminer l'emplacement de la projection sur l'image de l'endoscope.

3. Dispositif selon la revendication 2, dans lequel l'appareil de localisation de l'orifice (210) est une attache de détection de forme et l'appareil de positionnement et orientation est un stylet.

4. Dispositif selon la revendication 1, dans lequel l'appareil de localisation de l'orifice est au moins un élément rigide et l'appareil de positionnement et orientation est au moins un joint relié à l'au moins un élément rigide et ayant un codeur (280) adapté pour mesurer un angle du joint.

5. Système pour localiser un orifice pour un outil chirurgical par rapport à un endoscope, comprenant:
le dispositif de la revendication 1;
une mémoire (230) connectée de manière opérationnelle au processeur;
un endoscope (10) fournissant une image de l'endoscope (16); et
un programme d'instructions codé sur la mémoire et lorsqu'il est exécuté par le processeur, le programme d'instructions est adapté pour déterminer l'emplacement de l'orifice.

6. Système selon la revendication 5, dans lequel le programme d'instructions (232) exécuté par le processeur (220):
détermine l'emplacement de l'orifice dans un espace d'image de l'endoscope;
détermine une projection d'un instrument à travers l'emplacement de l'orifice sur une image provenant de l'endoscope; et
superpose une représentation de l'instrument sur l'image de l'endoscope correspondant à l'emplacement potentiel de l'orifice.

7. Système selon la revendication 6, comprenant en outre un appareil de positionnement et orientation (270) connecté de manière opérationnelle à l'appareil de localisation de l'orifice, l'appareil de positionnement et orientation étant adapté pour être manipulé pour fournir des angles de projection à l'emplacement de l'orifice pour une projection (216) d'un instrument sur l'image de l'endoscope (16), l'appareil de positionnement et orientation étant adapté pour capturer les angles de projection et étant adapté pour déterminer l'emplacement de la projection sur l'image de l'endoscope.

8. Système selon la revendication 7, dans lequel l'appareil de localisation de l'orifice (210) est une attache de détection de forme et l'appareil de positionnement et orientation est un stylet.

9. Système selon la revendication 7, dans lequel l'appareil de localisation de l'orifice (210) est au moins un élément rigide et l'appareil de positionnement et orientation est au moins un joint relié à l'au moins un élément rigide et ayant un codeur (280) adapté pour mesurer un angle du joint.

10. Produit de programme informatique comprenant un dispositif de stockage lisible par ordinateur (230) sur lequel est codé un programme d'instructions (232) exécutable par un processeur d'ordinateur (220) pour placer un orifice pour un outil chirurgical en fonction de données anatomiques en temps réel, le programme d'instructions comprenant:
des instructions de programme pour déterminer des données anatomiques en temps réel à partir d'une image provenant d'un endoscope; **caractérisé en ce que**
les instructions de programme sont adaptées pour utiliser l'appareil de localisation de l'orifice du dispositif de la revendication 1 pour identifier un emplacement optimal pour un orifice d'instrument par rapport à l'image provenant de l'endoscope,
le produit de programme informatique comprenant en outre:
des instructions de programme pour localiser un emplacement potentiel de l'orifice;
des instructions de programme pour déterminer une projection d'un instrument à travers l'emplacement potentiel de l'orifice sur le plan de l'image de l'endoscope;
des instructions de programme pour superposer une représentation de l'instrument sur l'image de l'endoscope correspondant à l'emplacement potentiel de l'orifice; et
des instructions de programme pour recevoir une indication indiquant si l'emplacement potentiel de l'orifice est ou non un emplacement d'orifice optimal;
où les instructions de programme sont codées sur le dispositif de stockage lisible par ordinateur.
